Europäisches Patentamt

**European Patent Office**

Office européen des brevets

(19)

(11) Publication number: **0 016 454**
**B1**

(12)     **EUROPEAN PATENT SPECIFICATION**

(45) Date of publication of patent specification: **08.02.84**     (51) Int. Cl.³: **A 61 B 5/00, G 01 K 11/16**

(21) Application number: **80101404.4**

(22) Date of filing: **18.03.80**

(54) **Apparatus for surveying the thermal topography of a surface, in particular of human skin.**

(30) Priority: **22.03.79 IT 335579**

(43) Date of publication of application:
**01.10.80 Bulletin 80/20**

(45) Publication of the grant of the patent:
**08.02.84 Bulletin 84/6**

(84) Designated Contracting States:
**AT BE CH DE FR GB NL SE**

(56) References cited:
**AU - A - 469 146**
**FR - A - 2 014 496**
**FR - A - 2 383 646**
**US - A - 3 533 399**
**US - A - 3 796 884**

(73) Proprietor: **Stremmenos, Cristos**
**Via Firenze, 4**
**I-40100 Bologna (IT)**
(73) Proprietor: **Koui, Maria**
**Via Focheas, 67**
**Patrasso (GR)**

(72) Inventor: **Stremmenos, Cristos**
**Via Firenze, 4**
**I-40100 Bologna (IT)**
Inventor: **Koui, Maria**
**Via Focheas, 67**
**Patrasso (GR)**

(74) Representative: **Modiano, Guido et al,**
**MODIANO & ASSOCIATI S.A.S. Via Meravigli, 16**
**I-20123 Milan (IT)**

Courier Press, Leamington Spa, England.

## Apparatus for surveying the thermal topography of a surface, in particular of human skin

This invention relates to an apparatus for surveying the thermal topography of a surface, in particular of human skin, wherein a layer of liquid crystals having cholesteric properties is applied in contact relationship to the area to be surveyed and then illuminated with a light in the visible spectrum range and photographed by means of a photographic camera mounted for adjustment with respect to the layer of liquid crystals.

It is known that the temperature of human skin is currently detected with the aid of mercury thermometers, thermocouples and thermistors.

All these instruments have the disadvantage of having a certain thermal capacity. Moreover, through their use, only the temperature of a small skin region can be measured, instead of a sufficiently large skin surface, to detect minimal temperature differences between one point and another of said surface, such as skin areas overlying veins and arteries which are slightly warmer than the adjacent areas, or in the pathological cases of subcutaneous tumoral tissues, which exhibit a slight temperature differential with respect to the adjoining ones.

To survey the temperature of larger areas, the use of cholesteric liquid crystals has already been proposed. As is known from the most authoritative studies, cholesteric liquid crystals may be represented as a superposition of layers of oriented molecules, which are oriented in each layer along a preferential vector such as to result in a helical structure having a certain pitch dimension.

Conventional temperature measuring methods exploit a phenomenon whereby the pitch of the helix depends on temperature, thereby it becomes possible, by letting a light beam to impinge on the liquid crystals, to obtain a selective reflection which is directly related to temperature. Thus, by coating a surface with a light reflective film of cholesteric liquid crystals, isochromatic zones (of same color) will appear on said surface at the isothermal zones (of same temperature). Also known is the use of mixtures of cholestric liquid crystals of different natures to obtain a good brilliance of the color. The application of the cholestric substance is carried out either directly or indirectly, following cladding or encapsulating in special supports.

Conventional techniques have some substantial disadvantages which affect their effectiveness. First of all, they have shortcomings as regards the quantitative evaluation of temperature owing to the difficult a achievement of a correlation between the reflected wavelength and temperature. Secondly, they provide no means of following ths evolution of a thermal contour, and no detailed definition of the warm areas can be obtained, especially in the case of a tumoural nucleus.

Devices are further known comprising a photographic camera for taking photographic reproductions of the chromatic image given by a layer of liquid crystals applied on the interested area.

An apparatus of this type is disclosed in the US patent No. 3.796.884. However the photograph of the interested area has only a prevalently qualitative character. In fact the colours do not permit to discriminate exactly the values of the temperatures at the various zone of the area, since the human eye is not able to appreciate chromatic differences due to differences of few tenths of degrees of temperature as for example it occurs in the pathological cases of subcutaneous tumours.

The US patent No. 3.533.399 discloses a method for measuring the temperature of human skin whereby interference filters are used which permit to increase the precision of the measurement. To this aim in the known method discharge lamps are used rich in wavelengths transmitted by the filters. However, the width of the transmission band is not less than ca. 10 millimicrons so that, considering temperature-wavelength diagrams for typical cholestric material at the temperature of human skin (36°—38°C), the measured temperature may vary by more than 1°C which is intolerable for diagnostic purposes.

A further temperature sensing device is disclosed in the Australian patent No. 469146 wherein filters are used which are constituted by cellophan sheets. Such filters are wide band filters so that only very approximate measurements may be achieved.

This invention sets out to provide an apparatus which enables the isothermal values of a surface, in particular a cutaneous one, to be determined quantitatively, thereby avoiding the drawbacks of the prior art.

The task of this invention is attained by an apparatus as defined in the appended claims.

Advantageously, the application of the liquid crystal layers is preceded by the coating of the area with a black substratum effective to absorb the light component which has passed through the liquid crystal.

The advantages offered by the invention are mainly in that the combination of a light source having a line spectrum with interference filters permits to restrict the transmission band to 2—3 millimicron thus achieving a greater precision of measurement. Furthermore the arrangement of the filters between the objective of the camera and the layer of liquid crystals avoids alteration of the image taken by the camera due to the ambient light.

Advantageously, one can utilize as line spectrum light, the light to be obtained from mercury-vapor lamps, or mercury-cadmium-zinc lamps, very rich in lines in the or mercury-

cadmium-zinc lamps, very rich in lines in the visible range (approximately 400—800 nm).

The separation of the various lines to obtain monochromatic light is effected by means of a series of interferential filters which have a maximum of transparency at the various wavelengths of the emission lines of the lamps being used. These filters may be placed in front of the film to be exposed in a progressive order with respect to the wavelengths with which one wishes to photograph the area to be surveyed, thereby, once the wavelength which is reflected by the liquid crystals covering the area being surveyed and which reaches the film through the filter is known, a reproduction is obtained of the isothermal contour of the area on the film, and a certain temperature may be attributed to the isochromatic image owing to the interrelation between the latter and the wavelength.

The various photographs, as a whole and as obtained with the various filters, may constitute a thermotopometric map of the area.

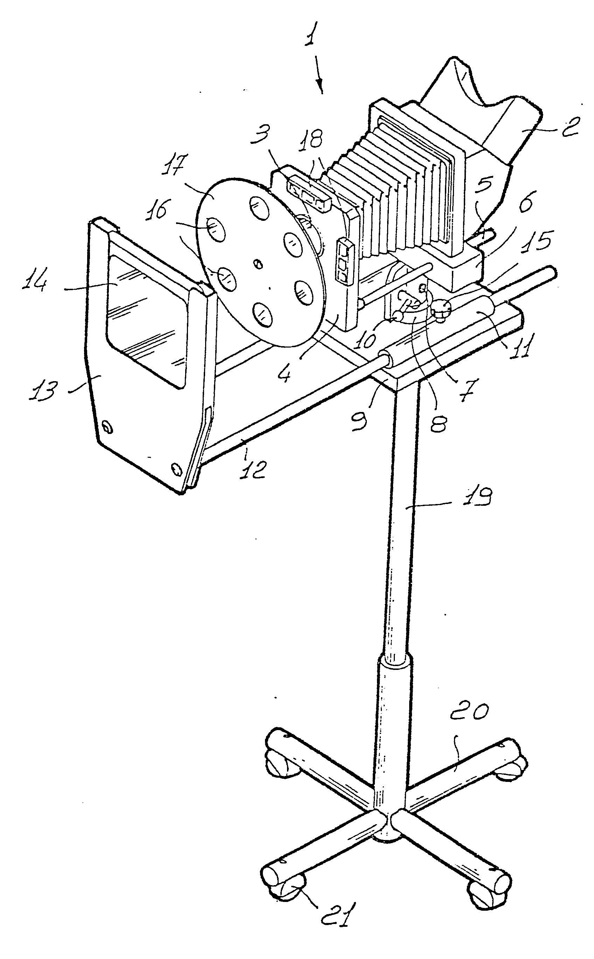

An embodiment of the invention is schematically illustrated in the accompanying drawing.

With reference to the drawing figure, the apparatus comprises a photographic camera, generally indicated at 1, which includes a visor 2, an objective 3 and a flange 4 supporting said objective. The flange 4 is provided with a pair of parallel rods 5 for adjusting the objective. The rods 5 are slidable in a supporting block 6, which is articulated at the lower end, on a horizontal axis, about a pivot pin 7 carried, cantilever-fashion, by a small base 8 attached to a plate 9. A specially provided lever 10, through a conventional threaded or cam type of locking device, positions the block 6 fixedly with respect to the plate 9, thus imparting to the camera the desired inclination.

With the plate 9, there are also rigidly associated two parallel sleeves 11 in which are guided respective stems 12, which support at one end a frame 13. In the frame, there is inserted a plate 14 carrying the liquid crystals and being located in front of the objective 3 at an appropriate distance therefrom. The position of the frame is fixed by securing the stems 12 within the sleeves 11 by means of screws 15.

Between the objective 3 and plate 14, there are interposed interferential filters 16 mounted on a disk 17 which is pivotable in an offset relationship to the camera 1 such that each filter can be rotated to reach a position exactly in front of the objective 3.

The reference numeral 18 designates mercury vapor flash lamps which are secured to the flange 4, either around the objective or sidewards thereof. Such lamps may be mercury vapor lamps manufactured by the Firm OSRAM and screened with a glass bulb to eliminate the ultraviolet radiation. The mercury lamps may be associated with other xenon lamps which afford the possibility of providing an overall chromatic display of the region being surveyed.

The whole apparatus is mounted on a column 19 which can be adjusted for height in a pedestal 20 equipped with caster wheels 21 for moving it around.

The operation of the apparatus will be next described with reference to the surveying of the temperature of a mammarian region. The patient is placed in front of the plate 14 with the region to be surveyed resting on said plate. Obviously, the mixture of liquid crystals will be selected as a function of the thermal range which includes the temperature of the mammarian skin. In this specific case, that range extends over 2°C on either side of an average value of 35°C. The mixture of cholesteric liquid crystals may be, for example, a 60% R and 40% L mixture, as suggested by 3M Company. (Instructions Liquid Crystal Kit Type 874), where R and L are standardized components.

Then, some photographs are shot, while interposing between the objective and plate a new interferential filter each time.

The photographs will reveal isothermal contours which are progressively narrower the shorter is the wavelength and the higher the temperature. The actual numerical value of the temperature can be obtained from a graph where the temperature is plotted as a function of the wavelength.

According to a preferred embodiment, the thermosensitive plate may be formed from a "Nylon" fabric for silk-screening (e.g. having a thickness dimension of 70 microns) onto which, by means of suitable mordants, there are anchored the black substratum or primer, with a water soluble pigments base, and a mixture comprising at least two microclad or microencapsulated cholesteric systems in appropriate ratios (e.g. 50% each, in the instance of two such systems).

These systems have the peculiarity of covering different but adjacent temperature ranges, with the obvious advantage that two or more isotherms can be obtained with one filter (i.e., with a single shot. This results in practice in an extension to the thermal range, with attendant improvement of the measuring sensitiveness (up to 1/10 of degree centigrade).

## Claims

1. An apparatus for surveying the thermal topography of a surface, in particular of human skin, whereon a layer (14) of liquid crystals having cholesteric properties, is applied in contact relationship to the area to be surveyed, the apparatus including a photographic camera (1) mounted for adjustment with respect to the layer (14) of liquid crystals characterized in that it comprises a light source (18) adapted for emitting a line spectrium light impinging said layer (14) of liquid crystals and at least one interference filter (16) interposable between the objective (3) of the camera (1) and said layer.

2. An apparatus according to claim 1 characterized in that said light source (18) is a mercury vapor flash lamp (18).

## Patentansprüche

1. Vorrichtung zur Überwachung der thermischen Topographie einer Oberfläche, insbesondere menschlicher Haut, auf der eine Schicht (14) aus flüssigen Kristallen mit cholesterischen Eigenschaften im Berührungsverhältniß mit dem zu überwachenden Flächenteil aufgebracht ist, wobei die Vorrichtung eine für die Einstellung in Bezug auf die Schicht (14) aus flüssigen Kristallen montierte photographische Kamera (1) aufweist, dadurch gekennzeichnet, daß sie eine Lichtquelle (18), die befähigt ist, ein auf die Schicht (14) aus flüssigen Kristallen einfallendes Licht mit Linienspektrum auszustrahlen, und mindestens einen zwischen dem Objektiv (3) der Kamera (1) und der genannten Schicht zwischenlegbaren Interferenzfilter (16) umfaßt.

2. Vorrichtung nach Anspruch 7, dadurch gekennzeichnet, daß die Lichtquelle (18) eine Quecksilberdampfblinklampe ist.

## Revendications

1. Dispositif de surveillance de la topographie thermique d'une surface, en particulier de la peau humaine, sur laquelle une couche (14) de cristaux liquides ayant des propriétés cholestériques est appliquée en contact avec la zone à surveiller, le dispositif comportant un appareil photographique (1) monté de façon à pouvoir être réglé par rapport à la couche (14) de cristaux liquides, caractérisé en ce qu'il comporte une source de lumière (18) adaptée pour émettre une lumière à spectre à lignes venant frapper cette couche (14) de cristaux liquides, et au moins un filtre d'interférences (16) pouvant être interposé entre l'objectif (3) de l'appareil photographique (1) et cette couche.

2. Dispositif selon la revendication 1, caractérisé en ce que cette source de lumière (18) est une lampe flash à vapeur de mercure (18).